# EUROPEAN PATENT SPECIFICATION

(11) **EP 0 650 701 B1**
(45) Date of publication and mention of the grant of the patent: **24.03.1999**
(21) Application number: 94307891.5
(22) Date of filing: 27.10.1994
(51) Int. Cl.: A61B 17/39

(54) **Electrosurgical apparatus for laporoscopy and similar interventions**
Elektrochirurgische Vorrichtung für die Laparascopie und ähnliche Verfahren
Appareil électrochirurgical pour laparoscopie et interventions similaires

(30) Priority: 01.11.1993 GB 9322464
(43) Date of publication of application: 03.05.1995
(73) Proprietor: GYRUS MEDICAL LIMITED, Cardiff CF3 0LX (GB)
(72) Inventor: Goble, Nigel Mark, Castleton, Nr. Cardiff CF3 8SB (GB); Goble, Colin Charles Owen, Penarth, South Glamorgan CF64 1AT (GB)
(74) Representative: Blatchford, William Michael

(56) References cited:
- US-A- 4 936 842
- US-A- 5 254 117
- US-A- 5 322 503
- US-A- 5 342 359
- US-A- 5 342 381

## Description

This invention relates to an electrosurgical instrument, particularly a laparoscopic instrument, and also to an electrode assembly for such an instrument.
It is known to construct an electrosurgical instrument in the form of a handpiece, a tubular shaft mounted in the handpiece and, at a distal end of the shaft, at least one fixed or movable electrode supplied with a radio frequency electrical potential via a conductor passing through the shaft and the handpiece. In an instrument for laparoscopic use in particular, it is advantageous to be able to rotate the electrode or electrodes about the axis of the shaft with respect to the handpiece. In addition, it is conventional to be able to remove and replace electrodes so that new electrodes are used for each surgical procedure. Generally, the remainder of the instrument is sterilisable and can be used for a number (typically 10) of operations before disposal. A known electrosurgical instrument of this type is described in US-A-4 936 842.

According to this invention, an improved electrode assembly for an electrosurgical instrument comprises an elongate tubular shaft mounting on a distal end portion thereof at least one electrode, an electrical conductor passing through the shaft and connected to the electrode, and, mounted on a proximal end portion of the shaft, mounting means for detachably mounting the assembly in a handpiece of the instrument, the mounting means comprising a first component shaped for attachment to the handpiece in a non-rotational relationship, and a second component secured to the shaft and rotatable with the shaft with respect to the first component about a longitudinal axis of the shaft, the conductor terminating in a contact portion housed in the first component and being so formed as to allow the electrode, the shaft, a part of the conductor in the distal end portion of the shaft, and the second component to rotate with respect to the first component and the contact portion about the longitudinal axis. This allows the assembly to be detachably mounted in the handpiece whilst permitting rotation of the shaft and electrode with respect to the handpiece. Preferably, the or each contact portion is fixed with respect to the first component whereby rotation of the shaft and electrode can be achieved without simultaneous sliding rotational contact between the contact portion and a supply conductor in the handpiece, giving improved reliability. Such an arrangement permits separate sterilisation of the handpiece which can, as a result, be used many more times than is customary with known instruments. In addition, the electrode assembly can be sterilised complete with electrodes, allowing multiple use. Consequently, the cost of electrosurgery can be reduced. Preferably, the conductor is flexible over at least part of its length, and may, indeed, be formed as a single metallic element which is flexible over substantially the whole of the length of the shaft.

For bipolar electrosurgery, the electrode assembly may have two electrodes mounted on the distal end portion of the shaft, and two respective flexible conductors inside the shaft insulated from one another and having two respective contact portions housed in the first component of the mounting means. The conductors may be elongate metallic strips, with each strip being formed from a resilient material such as spring steel with the or each contact portion integral with the respective strip.

The contact portion may be bent back in the distal direction with respect to a portion of the conductor extending through the shaft so as to lie on the outside of the first component of the mounting means for connection to an appropriately positioned contact in the handpiece. In the preferred embodiment of the invention, the second component of the mounting means is a sleeve fixed to the proximal end portion of the shaft, rotation control means being provided on the sleeve. In this case, the first component may comprise a tubular housing for the sleeve.

The tubular housing may include means for securing the end of the or each contact portion, and may have a longitudinal key feature such as a longitudinal key ridge or rail for engaging a corresponding key feature in the handpiece, allowing sliding insertion of the housing into the handpiece when the electrode assembly is being attached to the handpiece, but, at the same time, preventing rotation of the housing with respect to the handpiece. Consequently, the contact portion is held at a fixed rotational position with respect to a mating contact or contacts in the handpiece. A discontinuity may be provided in the form of an annular rail or groove in the interior surface of the housing for locating the sleeve inside longitudinally. Preferably, the housing is in at least two parts, one of the parts comprising a cover, the discontinuity being provided on the cover.

An irrigation fluid passageway in communication with the interior of the shaft may be provided in the second component to allow irrigation fluid to be pumped along the shaft, a flexible seal being situated at the proximal end of the shaft to close off the interior of the shaft at that end.

The invention also includes an electrosurgical instrument comprising a handpiece and an electrode assembly as described above, detachably mounted to the handpiece. The handpiece includes at least one electrical supply conductor which has a contact for sliding contact with a respective contact portion of the electrode assembly.

Electrical connection between the contact portion of the electrode assembly and the handpiece supply conductor contact is best achieved by sliding contact in the longitudinal or axial direction when the electrode assembly is inserted into or removed from the handpiece. Either the contact or the contact portion, or both, may be resiliently deflectable to allow for tolerances in dimensions of the handpiece or the assembly.

The invention will now be described by way of example with reference to the drawings, in which:-
Figure 1 is a partly sectioned side view of an electrosurgical instrument having an electrode assembly in accordance with the invention;
Figure 2 is a longitudinally sectioned plan view of a handpiece and a proximal part of the electrode assembly of the instrument of Figure 1;
Figure 3 is a transverse cross-section through the handpiece on the line III-III in Figure 1; and
Figure 4 is a perspective view of the proximal part of the electrode assembly.

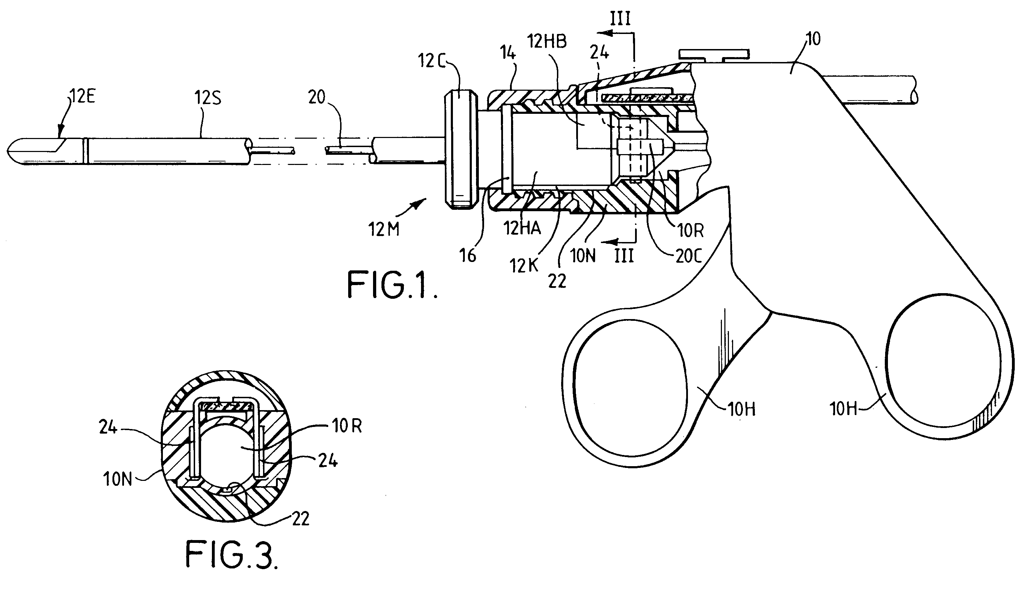

Referring to Figures 1 to 4, a laparoscopic electrosurgical instrument in accordance with the invention has a handpiece 10 with a hollow nose portion 10N providing a receptacle 10R for a detachable electrode assembly 12 which, in Figure 1, is shown mounted inside the handpiece receptacle 10R.

The electrode assembly comprises mounting means 12M received in the receptacle 10R and held in place by a screw-threaded retaining ring 14 which captures an annular ridge 16 on the outer surface of the mounting means 12M, the retaining ring being screw-threaded onto the outer cylindrical surface of the handpiece nose 10N.

The electrode assembly further comprises a tubular stainless steel shaft 12S, the proximal end portion of which is secured inside the mounting means 12M and, mounted on the distal end of the shaft 12S is a pair of electrodes 12E for bipolar electrosurgical cautery, for example.

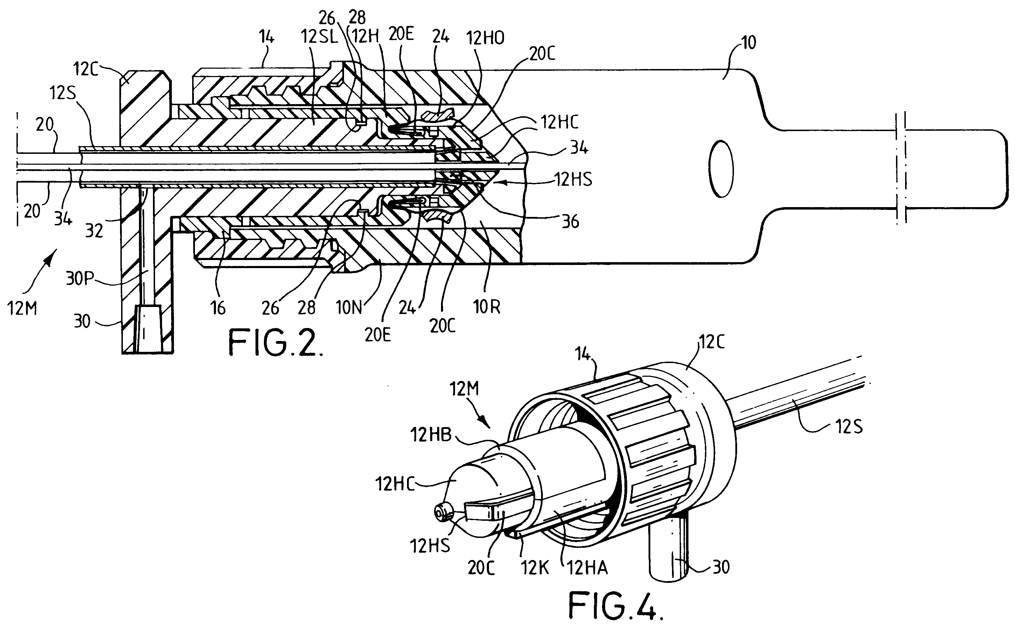

Running through the tubular shaft 12S is a pair of flexible electrical conductors 20, each of which extends from a respective electrode 12E to an integral spring steel contact portion 20C located on the outer surface of the mounting means 12M. In fact, the contact portions 20C are mounted on diametrically opposite sides of the mounting means 12M, and a longitudinal key rail 12K is integrally moulded on a lower surface of the mounting means 12M for engaging a keyway 22 in the receptacle 10R of the handpiece 10, this keyway being visible in the transverse cross-section of the handpiece nose in Figure 3. Due to upper positioning of the keyway 22, the contacts 20C, which are perpendicularly arranged with respect to the key rail 12K on the mounting means 12M, are received on opposite sides of the receptacle 10R where they engage vertically oriented metallic contact posts 24 housed in the handpiece nose 10N, as shown in Figures 2 and 3. Supply conductors (not shown) inside the handpiece 10 connect the contact posts 24 to a source of radio frequency electrical power.

The construction of the electrode assembly mounting means 12M allows the electrodes 12E, together with the tubular shaft 12S, to be rotated about the axis of the shaft relative to the handpiece 10 without disturbing the relative locations of the contact portions 20C and the contact posts 24.

Referring to Figure 2, the mounting means 12M have two main components. A first component comprises a tubular housing 12H, in the form of an elongate cap, which receives the second component constituted by a sleeve 12SL surrounding, and fixed to, the proximal end portion of the tubular shaft 12S. Both components are plastic mouldings, the sleeve 12SL being rotatable inside the housing 12H about the longitudinal axis of the shaft 12S, and having an annular groove 26 which receives an interior annular ridge 28 of the housing 12H to provide longitudinal location of the sleeve 12SL within the housing 12H. Since the sleeve 12SL is fixed to the shaft 12S, and since, as described above, the housing 12H is so mounted in the handpiece nose 10N that it cannot rotate with respect to the handpiece 10, rotation of the sleeve 12SL causes rotation of the shaft 12S and electrodes 12E with respect to the handpiece 10 about the axis of the shaft 12S. A collar portion 12C of the sleeve 12SL, visible in Figures 1, 2, and 4, is exposed beyond the end of the handpiece nose 10N when the electrode assembly 12 is mounted in the handpiece 10, where it can be gripped for rotating the electrodes 12E, as required, during use.

Incorporated in the rotation collar 12C is an integral pipe stub 30 (see Figure 2) with an inner passageway 30P in registry with an aperture 32 in the tubular shaft 12S to allow the supply of irrigation fluid to the interior of the tubular shaft 12S and thence to the region of the electrodes 12E.

Referring to Figure 4 in conjunction with Figure 1, the housing of the mounting means 12M is in two parts, the first part 12HA constituting the main part of the housing 12H extends the full length of the housing and includes the whole of the rotation collar 12C. In the region of the end of the mounting means 12M furthest from the electrodes, the main part 12HA is in the form of a half cylinder, the other half of the cylinder being formed by the second part 12HB acting as a cover which is a snap fit on the main part 12HA. As will be seen by comparing Figures 1 and 2, the annular ridge 28 for locating the sleeve 12SL is formed at a location where the main part 12HA and the cover part 12HB of the housing 12H form opposite walls of the housing 12H, so that part of the ridge 28 is formed by a feature of the cover part 12HB. This allows assembly of the mounting means 12M by placement of the sleeve 12SL within the main part 12HA of the housing 12H, followed by snap location of the cover part 12HB to secure the sleeve 12SL with its locating groove 26 housing the ridge 28, as shown in Figure 2.

Referring to Figures 1, 3 and 4, the electrical conductors 20 connected to the electrodes 12E are formed as stainless steel strips each with an insulative coating (not shown) to isolate the conductors from the shaft 12S and a control rod 34 passing through the shaft 12S for controlling movement of the electrodes 12E. At the end of the shaft 12S which is furthest from the electrodes 12E, the conductors pass through an elastomeric sealing cap 36 (e.g. made of silicone rubber) which is clamped over the end of the sleeve 12SL by the housing 12H to seal off the interior space of the proximal end portion of the tubular shaft 12S thereby to prevent escape of irrigation fluid supplied via passage 30P. This clamping is effected by the cap configuration of the housing 12H which has a generally conical cap end 12HC for guiding the electrode assembly into its receptacle in handpiece 10. As mentioned above, the contact portions 20C are arranged on the outside of the housing 12H in diametrically opposite positions, as shown in Figure 4. In fact, the contact portions 20C are integral parts of the conductors 20, the metallic strip in each case being exposed to the outside of the housing 12H insofar as the end portion of each conductor 20, here without an insulating covering, passes through a respective slot or aperture 12HS in the end cap of the housing 12H and is bent back in the distal direction over the outer surface of the housing 12H, the extreme end portion 20E of each strip being folded inwardly to run once again in the proximal direction in a cavity in the housing 12H, thereby securing the exposed contact portion 20C. By referring to Figure 4, it will be appreciated that the slots 12HS and the cavities housing the extreme end portions 20E of the conductors are formed in both parts 12HA and 12HB of the housing.

Each contact portion 20C is formed so that it stands away from the outer surface 12HO of the housing 12H over a portion of its length so as to be resiliently deflectable towards the surface 12HO by the contact posts 24 (see Figures 2 and 3) when the electrode assembly is inserted in the handpiece receptacle 10R.

The control rod 34 for the electrodes passes through the sealing cap 36 and the cap end of the housing 12H to be received within the handpiece 10.

It will now be appreciated that, to prepare the instrument for use, the electrode assembly 12, sterilised, is mounted in the handpiece 10 by pushing the mounting means 12M (Figure 1), with the retaining ring 14 in place around the annular ridge 16, into the receptacle 10R with the contact portions 20C facing opposite sides of the handpiece 10 so that the guide rail 12K engage the keyway 22 in the handpiece nose 10N. When the mounting means 12M is pushed fully home in the receptacle 10R, the contact posts 24 of the handpiece engage the contact portions 20C of the electrode assembly to form an electrical circuit between the supply conductors in the handpiece and the electrodes 12E. The assembly is secured in the handpiece by screwing the retaining ring 14 onto the threaded external surface of the handpiece nose 10N, as shown in Figure 2. Since the sleeve part 12SL of the mounting means 12M is rotatable within the housing part 12H, but is fixed to the tubular shaft 12S, rotation of the rotation collar 12C causes the electrodes 12E also to rotate, allowing the surgeon to orient the electrodes as required while keeping the handpiece in the most suitable orientation for manipulating the instrument, and operating the handles 10H. Since the conductors 20 are free to move inside the tubular shaft 12S, the electrodes 12E may be rotated about the axis of the shaft without rotation of the contact portions 20C. Consequently, no rotational sliding contact between the contact portions 20C and the contact posts 24 in the handpiece is necessary, with a consequent improved reliability of connection.

## Claims

1. An electrode assembly (12) for an electrosurgical instrument, the assembly comprising an elongate tubular shaft (12S) mounting on a distal end portion thereof at least one electrode (12E), an electrical conductor (20) passing through the shaft and connected to the electrode, and, mounted on a proximal end portion of the shaft, mounting means (12M) for detachably mounting the assembly to a handpiece (10) of the instrument, the mounting means comprising a first component (12H) shaped for attachment to the handpiece in a non-rotational relationship, and a second component (12SL) secured to the shaft and rotatable with the shaft with respect to the first component about a longitudinal axis of the shaft, the conductor terminating in a contact portion (20C) housed in the first component and being formed so as to allow the electrode, the shaft, a part of the conductor in the distal end portion of the shaft, and the second component to rotate with respect to the first component and the contact portion about the longitudinal axis.

2. An assembly according to claim 1, having two electrodes (12E) mounted on the distal end portion of the shaft (12S), and two respective insulated conductors (20) inside the shaft insulated from one another and having two respective contact portions (20C) housed in the first component (12H) of the mounting means (12M).

3. An assembly according to claim 1 or claim 2, wherein the or each conductor (20) is formed as an elongate metallic strip.

4. An assembly according to claim 3, wherein the or each contact portion (20C) is integral with the respective strip (20).

5. An assembly according to claim 3 or claim 4, wherein the or each contact portion (20C) is bent back in the distal direction with respect to a portion of the conductor (20) extending through the shaft (12S).

6. An assembly according to any preceding claim, wherein the second component (12SL) comprises a sleeve fixed to the proximal end portion of the shaft (12S) and rotation control means on the sleeve, and the first component (12H) comprises a tubular housing for the sleeve.

7. An assembly according to claim 6, wherein the tubular housing includes means for securing the end of the or each contact portion (20C).

8. An assembly according to claim 6 or claim 7, wherein the tubular housing includes a key feature (16) for allowing slidable insertion of the housing into the handpiece (10) preventing rotation with respect to the handpiece.

9. An assembly according to any of claims 6 to 8, wherein the tubular housing has a discontinuity (28) for longitudinal location of the housing with respect to the second component (12SL)

10. An assembly according to claim 9, wherein the housing has a cover and the discontinuity (28) is on the cover.

11. An assembly according to any of claims 6 to 10, wherein the second component (12SL) includes an irrigation fluid passageway (30P) in communication with the interior of the shaft (12S) and wherein the assembly (12) includes a flexible seal (36) arranged to close the interior of the distal end portion of the shaft.

12. An electrosurgical instrument comprising a handpiece (10) and an electrode assembly (12) as defined in any preceding claim detachably mounted to the handpiece, wherein the handpiece includes at least one electrical supply conductor which has a contact (24) for sliding contact with a respective contact portion (20C) of the assembly.

13. An instrument according to claim 12, wherein the or each handpiece supply conductor contact (24) and the or each electrode assembly contact portion (20C) are arranged for relative sliding contact with each other in an axial direction, either the or each handpiece contact or the or each electrode assembly contact portion being resiliently deflectable.

## Patentansprüche

1. Elektrodenanordnung (12) für ein elektrochirurgisches Instrument, mit: einem länglichen, rohrförmigen Schaft (12S), an dessen distalem Endteil mindestens eine Elektrode (12E) montiert ist; einem elektrischen Leiter (20), der durch den Schaft läuft und mit der Elektrode verbunden ist und am proximalen Endteil des Schafts montiert ist; einer Montageeinrichtung (12M) zum abnehmbaren Anbringen der Anordnung an einem Handteil (10) des Instruments, die eine erste Komponente (12H), die für Befestigung am Handteil in nicht verdrehbarer Beziehung geformt ist, und eine zweite Komponente (12SL) aufweist, die am Schaft befestigt ist und mit dem Schaft in Bezug auf die erste Komponente um die Längsachse des Schafts verdrehbar ist; wobei der Leiter in einem in der ersten Komponente untergebrachten Kontaktteil (20C) endet und so ausgebildet ist, dass er es der Elektrode, dem Schaft, einem Teil des Leiters im distalen Endteil des Schafts sowie der zweiten Komponente ermöglicht, sich in Bezug auf die erste Komponente und den Kontaktteil um die Längsachse zu drehen.

2. Anordnung nach Anspruch 1, mit zwei Elektroden (12E), die am distalen Endteil des Schafts (12S) angebracht sind, und mit zwei jeweiligen isolierten Leitern (20) innerhalb des Schafts, die gegeneinander isoliert sind und zwei jeweilige Kontaktteile (20C) aufweisen, die in der ersten Komponente (12H) der Montageeinrichtung (12M) untergebracht sind.

3. Anordnung nach Anspruch 1 oder Anspruch 2, bei der der Leiter (20), oder jeder Leiter, als langgestrecktes Metallband ausgebildet ist.

4. Anordnung nach Anspruch 3, bei der der Kontaktteil (20C), oder jeder Kontaktteil, einstückig mit dem jeweiligen Band (20) ausgebildet ist.

5. Anordnung nach Anspruch 3 oder Anspruch 4, bei der der Kontaktteil (20C), oder jeder Kontaktteil, in distaler Richtung in Bezug auf einen Teil des sich durch den Schaft (12S) erstreckenden Leiters (20) zurückgebogen ist.

6. Anordnung nach einem der vorstehenden Ansprüche, bei der die zweite Komponente (12SL) eine am proximalen Endteil des Schafts (12S) befestigte Hülse sowie eine Dreheinstelleinrichtung an der Hülse aufweist, und die erste Komponente (12H) ein rohrförmiges Gehäuse für die Hülse aufweist.

7. Anordnung nach Anspruch 6, bei der das rohrförmige Gehäuse eine Einrichtung zum Befestigen des Endes des Kontaktteils (12C), oder jedes Kontaktteils, aufweist.

8. Anordnung nach Anspruch 6 oder Anspruch 7, bei der das rohrförmige Gehäuse ein als Schlüssel wirkendes Merkmal (16) aufweist, das ein verschiebbares Einführen des Gehäuses in das Handteil (10) unter Vermeidung einer Verdrehung in Bezug auf das Handteil ermöglicht.

9. Anordnung nach einem der Ansprüche 6 bis 8, bei der das rohrförmige Gehäuse eine Unstetigkeit (28) für Positionierung des Gehäuses in Längsrichtung in Bezug auf die zweite Komponente (12SL) aufweist.

10. Anordnung nach Anspruch 9, bei der das Gehäuse eine Abdeckung aufweist und sich die Unstetigkeit (28) an dieser befindet.

11. Anordnung nach einem der Ansprüche 6 bis 10, bei der die zweite Komponente (12SL) einen Spülfluidkanal (30P) in Verbindung mit dem Inneren des Schafts (12S) aufweist, und wobei die Anordnung (12) eine flexible Abdichtung (36) aufweist, die so angeordnet ist, dass sie das Innere des distalen Endteils des Schafts verschließt.

12. Elektrochirurgisches Instrument mit einem Handteil (10) und einer Elektrodenanordnung (12) nach einem der vorstehenden Ansprüche, das abnehmbar am Handteil angebracht ist, wobei das Handteil mindestens einen elektrischen Versorgungsleiter aufweist, der einen Kontakt (24) für Gleitkontakt zu einem jeweiligen Kontaktteil (20C) der Anordnung aufweist.

13. Instrument nach Anspruch 12, bei dem der Versorgungsleiterkontakt (24), oder jeder Versorgungsleiterkontakt, des Handteils sowie der Kontaktteil (20C), oder jeder Kontaktteil, der Elektrodenanordnung für relativen, gegenseitigen Gleitkontakt in axialer Richtung ausgebildet sind, wobei entweder der Handteilkontakt, oder jeder Handteilkontakt, oder der Elektrodenanordnungs-Kontaktteil, oder Elektrodenanordnungs-Kontaktteil, nachgiebig auslenkbar sind.

## Revendications

1. Module d'électrode (12) pour appareil électrochirurgical, comprenant un arbre tubulaire allongé (12S) possédant au moins une électrode (12E) montée dans la partie d'extrémité distale de ce dernier, un conducteur électrique (20) traversant l'arbre et relié à l'électrode, et, sur la partie d'extrémité proximale de l'arbre, des moyens de montage (12M) aptes à permettre le montage de manière amovible du module sur une pièce à main (10) de l'instrument, caractérisé en ce que les moyens de montage consistent en un premier composant (12H) conformé pour être fixé solidairement à la pièce à main, et un second composant (12SL) solidarisé à l'arbre et monté à rotation avec ce dernier par rapport au premier composant autour d'un axe longitudinal de l'arbre ; le conducteur électrique se terminant sur une pièce de contact (20C) logée dans le premier composant et conformée de manière à permettre à l'électrode, à l'arbre, à une partie du conducteur dans la zone d'extrémité distale de l'arbre, et au second composant de tourner par rapport au premier élément et à la pièce de contact autour de l'axe longitudinal.

2. Module d'électrode selon la revendication 1, caractérisé en ce qu'il présente deux électrodes (12E) montées dans la zone d'extrémité distale de l'arbre (12S) et deux conducteurs (20) respectifs isolés l'un de l'autre à l'intérieur de l'arbre et se terminant respectivement sur deux pièces de contact (20C) logées dans le premier composant (12H) des moyens de montage (12M).

3. Module d'électrode selon l'une des revendications 1 ou 2, caractérisé en ce que le ou les conducteurs (20) sont constitués d'une bande métallique allongée.

4. Module d'électrode selon la revendication 3, caractérisé en ce que le ou les pièces de contact (20C) sont respectivement parties intégrantes des bandes (20).

5. Module d'électrode selon l'une des revendications 3 ou 4, caractérisé en ce que la ou chaque pièce de contact (20C) est repliée dans la direction distale par rapport à une portion du conducteur (20) qui s'étend à travers l'arbre (12S).

6. Module d'électrode selon l'une quelconque des revendications précédentes, caractérisé en ce que le second composant (12SL) comprend un manchon fixé à la partie d'extrémité proximale de l'arbre (12S) et des moyens de contrôle du manchon en rotation, le premier composant (12H) comprenant un logement tubulaire pour le manchon.

7. Module d'électrode selon la revendication 6, caractérisé en ce que le logement tubulaire comprend des moyens de fixation de l'extrémité de la ou de chaque pièce de contact (20C).

8. Module d'électrode selon l'une des revendications 6 ou 7, caractérisé en ce que le logement tubulaire comprend une cannelure (16) permettant au logement d'être coulissé à l'intérieur de la pièce à main (10) en empêchant sa rotation par rapport à cette dernière.

9. Module d'électrode selon l'une quelconque des revendications 6 à 8, caractérisé en ce que le logement tubulaire présente une discontinuité (28) permettant de situer sur l'axe longitudinal le logement par rapport au second composant (12SL).

10. Module d'électrode selon la revendication 9, caractérisé en ce que le logement possède une protection et en ce que la discontinuité (28) est située sur cette protection.

11. Module d'électrode selon l'une quelconque des revendications 6 à 10, caractérisé en ce que le second composant (12SL) présente un passage (30P) d'irrigation pour un fluide en communication avec l'intérieur de l'arbre (12S), le module (12) comprenant un joint flexible (36) apte à fermer l'intérieur de la zone d'extrémité distale de l'arbre.

12. Appareil électrochirurgical comprenant une pièce à main (10) et un module d'électrode (12) tel que défini dans l'une quelconque des revendications précédentes, pouvant être monté sur et démonté de la pièce à main, caractérisé en ce que la pièce à main comprend au moins un conducteur électrique souple présentant un contact (24) apte à établir un contact flexible avec une des pièces de contact respectives (20C) du module d'électrode.

13. Appareil selon la revendication 12, caractérisé en ce que le ou chaque contact conducteur souple (24) de la pièce à main et la ou chaque pièce de contact (20C) du module d'électrode sont prévus pour établir un contact glissant mutuel dans une direction axiale, le ou chaque contact de la pièce à main ou la ou chaque pièce de contact du module d'électrode pouvant être fléchi élastiquement.
